# EUROPEAN PATENT SPECIFICATION

(11) **EP 1 360 484 B1**
(45) Date of publication and mention of the grant of the patent: **29.10.2008**
(21) Application number: 01912101.1
(22) Date of filing: 15.02.2001
(51) Int. Cl.: G01N 33/50, C12Q 1/34, C12Q 1/37

(54) **ENZYMATIC TEST FOR THE DETERMINATION OF THE RISK OF PATHOLOGIES RELATED TO THE PRESENCE OF SIALIDASE OR PROLIDASE ACTIVITY IN WOMEN BODY FLUID SAMPLES**
ENZYMTEST ZUR BESTIMMUNG VON KRANKHEITSRISIKEN IN VERBINDUNG MIT DER ANWESENHEIT VON SIALIDASE ODER PROLIDASE AKTIVITÄT IN KÖRPERFLÜSSIGKEIT VON FRAUEN
EPREUVE ENZYMATIQUE PERMETTANT DE DETERMINER LE RISQUE DE PATHOLOGIES ASSOCIEES A LA PRESENCE D'UNE ACTIVITE SIALIDASE OU PROLIDASE DANS DES ECHANTILLONS DE FLUIDES CORPORELS FEMININS

(43) Date of publication of application: 12.11.2003
(73) Proprietor: UNIBIO S.r.l., 40125 Bologna (IT)
(72) Inventor: CAUCI, Sabina, I-34074 Monfalcone (IT)
(74) Representative: Crippa, Paolo Ernesto
(86) International application number: PCT/IT2001/000069
(87) International publication number: WO 2002/065122

(56) References cited:
- WO-A-00/24753
- WO-A-00/55354
- US-A- 5 571 684
- MCGREGOR J A ET AL: "BACTERIAL VAGINOSIS IS ASSOCIATED WITH PREMATURITY AND VAGINAL FLUID MUCINASE AND SIALIDASE: RESULTS OF A CONTROLLED TRIAL OF TOPICAL CLINDAMYCIN CREAM" AMERICAN JOURNAL OF OBSTETRICS & GYNECOLOGY, MOSBY, ST LOUIS, MO, US, vol. 170, no. 4, April 1994 (1994-04), pages 1048-1060, XP002901155 ISSN: 0002-9378
- BRISELDEN A M ET AL: "SIALIDASES (NEURAMINIDASES) IN BACTERIAL VAGINOSIS AND BACTERIAL VAGINOSIS-ASSOCIATED MICROFLORA" JOURNAL OF CLINICAL MICROBIOLOGY, WASHINGTON, DC, US, vol. 30, no. 3, March 1992 (1992-03), pages 663-666, XP002901154 ISSN: 0095-1137
- DATABASE ONLINE MEDICAL DICTIONARY University of Newcastle upon Tyne; 12 March 2000 (2000-03-12), "PID"
- BARBARA AND MAJERONI: "Bacterial vaginosis: an update" AMERICAN FAMILY PHYSICIAN, 15 March 1998 (1998-03-15),
- www.cdc.gov/std/bv/stdfact-bacterial-vagin osis.htm
- Pubmed abstract of Myer et al, Lancet Infect Dis, 2005, Dec, 5(12):786
- Pubmed abstract of Myer et al, J Infect Dis, 2005 Oct 15, 192(8): 1372-80
- Pubmed abstract of Schwebke, J Infect Dis, 2005 Oct 15, 192(8):;1315-7
- Pubmed abstract of Hay, Curr Opin Infect Dis, 2004 feb, 17(1): 41-4
- Pubmed abstract of Wiesenfeld et al, Obstet Gynacol 2002 sep, 100(3):456-63
- Pubmed abstract of Wilson et al, BJOG 2002 jun, 109(6): 714-7

## Description

The current invention describes a method for the determination of the risk of pathologies related to sialidase and/or prolidase activity in samples of vaginal fluid of women, wherein said pathologies are low birth weight (LBW), preterm delivery (PTD), preterm rupture of membranes, intraamniotic infections, spontaneous abortion, endometritis, post-partum or post-gynecologic surgery infections, upper genital tract infections (PID) which cause infertility, cervicitis, susceptibility to sexually transmitted diseases as viral infections from papillomavirus (HPV) and human immunodeficiency virus (HIV), and vertical transmission of HIV infection.

Low birth weight (LBW), primarily due to preterm birth (PTD), is the main risk factor for neonatal morbidity, mortality and long term infant neurological disorders.

Most cases of low birth weight have no recognized causal factors, making current prevention strategies ineffective. However, several investigations have shown that bacterial vaginosis (BV) is associated with preterm delivery (PTD), with increased relative risk from 1.4 to 6.9 fold in respect to absence of BV, preterm delivery of low-weight infants, early and late miscarriage, and in general with maternal complications. Moreover, recently bacterial vaginosis was associated with increased risk of sexual acquisition of the viral infection by HIV and of vertical mother-to-child HIV transmission. Bacterial vaginosis prevalence is about 10% in non-pregnant women and about 15% in pregnant women in Western Europe, from 10 to 30% in US pregnant women, over 30% in colored African origin women, over 60% in women attending sexually transmitted disease clinics. Anyway, it is to be noted that only a small percentage of women with BV have adverse pregnancy outcomes or acquire HIV infection, among subjects at elevated risk of HIV viral exposure, so there is the need to have selective markers to individuate patients at elevated risk who need to be therapeutically treated.

Bacterial vaginosis is caused by different bacterial species comprising *G. vaginalis, Bacteroides* spp., *Prevotella* spp. and *Mobiluncus* spp., *Ureaplasma urealyticum* and *Mycoplasma hominis.* The pathogenesis of bacterial vaginosis is still obscure and not always the presence of such pathologic condition is easy to diagnose.

In particular, it is not known what triggers the shift from the normal lactobacilli colonization to the altered vaginal flora, or which degree of the vaginal microbial flora alteration is indicative of a real pathologic condition.

A crucial issue in bacterial vaginosis is the synergy between *G*. *vaginalis* and anaerobic bacteria.

*G. vaginalis* is found in high titers in over the 95% of bacterial vaginosis cases, whereas low titers of *G. vaginalis* colonization are found also in women without bacterial vaginosis.

However, a strict correlation between *G. vaginalis* colonization and the condition of bacterial vaginosis has been demonstrated.

The only fully characterized virulence factor released by *G. vaginalis* is a β-hemolytic toxin (Gvh) having a molecular weight of about 59,000 Dalton.

Furthermore, it has been shown that about half of the non-pregnant women develop an immune response against Gvh eliciting IgA antibodies at the vaginal mucosa level. The absence of anti-Gvh IgA antibodies in women with BV is mainly due to the inactivation of IgA and this phenomenon is related to the presence of high levels of vaginal sialidase activity. Such inactivation derives from a structural impairment of IgA likely due to the hydrolytic action of an array of factors released by anaerobic bacteria.

American Journal of Obstetrics & Gynecology (vol. 170, no. 4, April 1994, pages 1048-1060) discloses a study performed on the comparison between women with and without bacterial vaginosis, these last after having been treated with clindamycin, to see whether pregnancy outcomes could be improved analysing pregnancy results and changes in vaginal fluid mucinase and sialidase after treatment with 2% clindamycin vaginal cream for bacterial vaginosis.

WO 0055354 discloses a diagnostic test for diseases such as BV which detects the presence of sialidase activity. No reference to a method for the determination of the risk of pathologies related to the presence of bacteria producing sialidase and/or prolidase is made.

WO 0024753 discloses a method of diagnosis of sialidase related disease using a particular substrate compound. No reference to a method for the determination of the risk of pathologies related to the presence of bacteria producing sialidase and/or prolidase is made.

Presently it is not known which factors of the antimicrobial host defense are compromised or which microbial virulence factor(s) elicits the IgA cleavage in a subgroup of women with bacterial vaginosis.

Above all, it is not yet established if this condition is associated with increased risk of BV complications.

Such an uncertain definition makers clinicians to face the problem whether or not administer a pharmacological therapy to women with bacterial vaginosis. It is well known that two different drugs are used to treat bacterial vaginosis: metronidazole or clindamycin. Both these drugs show a main drawback consisting in relevant adverse side effects, in particular metronidazole can give gastrointestinal symptoms, and is considered a teratogen agent, so it is not recommended during pregnancy, on the other hand clindamycin can cause diarrhea and psendomembranous colitis which may be lethal. Moreover both such antibiotics can cause yeast vaginitis.

For the above described reasons it is highly desirable the development of a method to determine the risk of pathologic complications as, for example, low birth weight (LBW), preterm birth (PTD), spontaneous abortion, premature rupture of membrane, endometritis, acquisition of HIV infection, and of HPV infection (which causes cervical intraepithelial neoplasia), upper genital tract infections (PID), post-partum and post-gynecologic surgery infections.

The present invention provides a reliable method to solve the problem of the risk determination of the above-described complications by furnishing the physician a valuable tool to decide whether or not administer a pharmacological therapy.

Such a problem is solved by a method for determination of risk of pathologies as expressed in the main enclosed claim.

Further features and advantages of the method of the present invention will be highlighted by the subsequent description of preferred embodiments, given at indicative and not limitative title.

For the setting up of the method of the current invention clinical cases have been studied and compared with controls of women with no recognized pathologic conditions.

In particular, 116 women who had low birth weight babies (LBW), 86 women who had a preterm delivery (PTD), and 417 women who had normal birth weight babies at term of gestation (NTD) were examined.

The presence of sialidase and/or prolidase activity was detected in the vaginal fluid samples of all these women, moreover the levels of said sialidase and prolidase activity were evaluated.

Sialidases are enzymes produced by bacteria and are involved in the pathogenesis of several diseases. These enzymes provoke the hydrolysis of sialic acid from various glycoproteins including IgA, altering the immune response.

Prolidases are proteolytic enzymes produced by bacteria to favor the cell infiltration. These enzymes are able to activate cytokines and other immune mediators.

The sialidase activity was determined by incubation of 50 µl of the vaginal fluid sample with 50 µl of a substrate at pH 5.0, with a procedure described by Cauci et al., Am J Obstet Gynecol 1998; 178: 511-5. Specific activity was expressed as nanomoles of methoxyphenol produced from conversion of the substrate and calculated by comparison with a standard curve of pure methoxyphenol. The levels of sialidase activity were evaluated as: no activity for values below 0.19 nmol of methoxyphenol, +1 cutoff for values equal or above 0.19 nmol of methoxyphenol; +2 cutoff for values equal or above 0.38 nmol of methoxyphenol; +3 cutoff for values equal or above 2.50 nmol of methoxyphenol, and +4 cutoff for values equal or above 5.0 nmol of methoxyphenol.

Prolidase activity was determined as described by Cauci et al., J Infect Dis 1998; 178:1698-706. The levels of prolidase activity were evaluated as: no activity for values below 22 mOD, +1 cutoff for values equal or above 22 mOD; +2 cutoff for values equal or above 44 mOD; +3 cutoff for values equal or above 1000 mOD and +4 cutoff for values equal or above 2000 mOD.

The +1 cutoffs of sialidase and prolidase activity were calculated by the mean value plus one standard deviation measured in a healthy control population (that is devoid of any vaginal symptom and with no microbial vaginal colonization except lactobacilli). The +2 cutoff was obtained by doubling the +1 cutoff. The +3 cutoff was obtained by halving the +4 cutoff.

In particular, to set up the method of this invention said values of sialidase and prolidase activity were associated with LBW and PTD.

The following Table 1 shows data analyzed as percentage of women which had low birth weight babies (LBW), preterm delivery (PTD) or normal term delivery (NTD, controls), whereas data in bold show the ratio (odds ratio, OR) between the percentage of women with LBW or PTD and the percentage of NTD women, respectively. This value was calculated and corrected by a standard factor by the SPSS computer statistic program.

In particular, the vaginal fluid samples were obtained from women in the first or in the second trimester of gestational age, and, preferably, in the period ranging from the seventh to the twenty-fourth full week of gestation.

These groups of women have been further subdivided on the basis of the presence, in the vaginal fluids samples, of sialidase activity alone, of prolidase activity alone, of these enzymatic activities and a pH ≥ 4.7, of these enzymatic activities and the pathologic condition bacterial vaginosis (BV), or of these enzymatic activities and the *G. vaginalis* bacterium.

**TABLE 1**

| | Positive % **OR** | | | | |
|---|---|---|---|---|---|
| **Measured parameter** **and/or vaginal colonization** | **NTD** **n=417** | **LBW** **n=116** | | **PTD** **n=86** | |
| **Sialidase activity** | | | | | |
| ≥ +1 | 15.3 | 28.7 | **2.2** | 20.0 | **1.4** |
| ≥ +2 | 10.3 | 18.3 | **1.9** | 11.8 | **1.2** |
| ≥ +3 | 3.8 | 7.8 | **2.1** | 4.7 | **1.2** |
| ≥ +4 | 1.0 | 3.5 | 3.7 | 2.4 | **2.5** |
| **Prolidase activity** | | | | | |
| ≥ +1 | 33.7 | 41.6 | **1.4** | 34.9 | **1.1** |
| ≥ +2 | 26.0 | 37.2 | **1.7** | 24.1 | **0.9** |
| ≥ +3 | 3.9 | 7.1 | **1.9** | 6.0 | **1.6** |
| ≥ +4 | 1.0 | 4.4 | **4.8** | 3.6 | **3.9** |
| **pH ≥ 4.7** | 30.5 | 37.1 | **13** | 32.6 | **1.1** |
| **pH ≥ 4.7 and Sialidase activity** | | | | | |
| ≥ +1 | 9.8 | 19.0 | **2.0** | 11.6 | **1.2** |
| ≥ +2 | 7.9 | 13.8 | 1.8 | 10.5 | **1.4** |
| ≥ +3 | 3.8 | 7.8 | **2.1** | 4.7 | **1.2** |
| ≥ +4 | 1.0 | 3.4 | **3.6** | 2.3 | **2.4** |
| **pH ≥ 4.7 and Prolidase activity** | | | | | |
| ≥ +1 | 20.9 | 31.0 | **1.6** | 25.6 | **1.3** |
| ≥ +2 | 18.5 | 30.2 | **1.7** | 20.9 | **1.2** |
| ≥ +3 | 3.6 | 6.0 | **1.8** | 4.7 | **1.4** |
| ≥ +4 | 0.7 | 3.4 | **5.1** | 2.3 | **3.5** |
| **BV** | 14.6 | 20.7 | **1.5** | 12.8 | **0.9** |
| **BV and Sialidase activity** | | | | | |
| ≥ +1 | 9.1 | 15.7 | **1.9** | 7.1 | **0.8** |
| ≥ +2 | 7.4 | 12.2 | **1.7** | 7.1 | **0.9** |
| ≥ +3 | 3.8 | 7.8 | **2.1** | 4.7 | **1.2** |
| ≥ +4 | 1.0 | 3.5 | **3.7** | 2.4 | **2.5** |
| **BV and Prolidase activity** | | | | | |
| ≥ +1 | 13.5 | 18.6 | **1.5** | 12.0 | **0.9** |
| ≥ +2 | 13.0 | 18.6 | **1.5** | 10.8 | **0.8** |
| ≥ +3 | 2.7 | 3.5 | **1.3** | 2.4 | **0.9** |
| ≥ +4 | 0.2 | 2.7 | **11.3** | 1.2 | **5.0** |
| ***G. vaginalis*** | 36.2 | 42.2 | **1.3** | 36.0 | **1.0** |
| ***G. vaginalis* and Sialidase activity** | | | | | |
| ≥ +1 | 11.0 | 18.3 | **1.8** | 11.8 | **1.1** |
| ≥ +2 | 8.2 | 13.0 | **1.7** | 9.4 | **1.2** |
| ≥ +3 | 3.8 | 7.8 | **2.1** | 4.7 | **1.3** |
| ≥ +4 | 1.0 | 3.5 | **3.7** | 2.4 | **2.5** |
| ***G. vaginalis* and Prolidase activity** | | | | | |
| ≥ +1 | 23.4 | 31.0 | **1.5** | 26.5 | **1.2** |
| ≥ +2 | 21.0 | 30.1 | **1.7** | 22.9 | **1.1** |
| ≥ +3 | 3.6 | 7.1 | **2.0** | 6.0 | **1.7** |
| ≥ +4 | 0.7 | 4.4 | **6.4** | 3.6 | **5.2** |

The main result shown in Table 1 is the OR value as it represents the index of risk of low birth weight (LBW) or of preterm birth (PTD).

If this ratio is lower than 1 it means that the woman has no or low risk of complications. A ratio ranging from 1 to 2 is associated with a medium risk of complications. If this ratio is comprised between 2 and 4 it means that the woman has a high risk of complications. Finally, if this ratio is higher than 4 it means that the woman has a very high risk of low birth weight or preterm birth.

In particular, and at title of example, a medium risk corresponding to an OR value equal 2 means that the patient has a twofold risk of complications compared to a normal woman, that is, in other words, a risk 100% higher.

Preferably, this result can be point out with a score: - meaning a low or no risk, + meaning a medium risk, ++ meaning a high risk, and +++ meaning a very high risk.

Table 1 highlights that the OR value has an essentially increasing profile in relation to the increase of the levels of sialidase or prolidase activity.

In particular, for levels of sialidase activity from +1 to +3 cutoffs the OR values for LBW are comprised between 2 and 4, whereas the OR values for PTD are comprised between 1 and 2. Consequently, said levels of sialidase activity in vaginal fluid samples, regardless to the BV condition, and regardless to the presence of *Gardnerella vaginalis,* are indicative of a high risk for LBW and a medium risk for PTD.

The OR values are in the range from 2 and 4 both for LBW and PTD for levels of sialidase activity equal or higher than +4. Consequently, the risk for LBW or PTD is high.

Likewise, for levels of prolidase activity from +1 to +3 cutoffs the OR values are comprised between 1 and 2, both for LBW and PTD. Therefore, the risk for LBW and PTD is medium.

On the other hand, for levels of prolidase activity equal or higher than +4, the OR value is higher than 4 for LBW and comprised between 2 and 4 for PTD. Consequently, the risk for LBW is very high, whereas the risk for PTD is medium.

From the analysis of the clinical data shown in Table 1, it is possible to relate the risk for LBW or PTD with the levels of sialidase and/or prolidase activity detected in the body fluid of the patient, preferably in a sample of vaginal fluid.

On the grounds of the results just shown, and according to the current invention, a method has been set up for the determination of the risk of pathologies related to the presence of sialidase or prolidase activity in samples of body fluid, as low birth weight (LBW), preterm birth (PTD), premature rupture of membranes, intraamniotic infections, spontaneous abortion, endometritis, post-partum and post-gynecologic surgery infections, upper genital tract infections (PID) which cause infertility, cervicitis, susceptibility to sexually transmitted diseases as viral infections from papillomavirus (HPV) and human immunodeficiency virus (HIV), and vertical transmission of HIV infection.

Said method comprises the following steps in the order:
a) determination of levels of sialidase and/or prolidase activity in said body fluid sample;
b) comparison of such levels of sialidase and/or prolidase activity with prefixed ranges of said activities.
c) calculation of the risk factor.

It is so evident that an effective method has been devised to evaluate the risk of pathologies related to the presence of sialidase and/or prolidase activity in samples of body fluid of women. Profitably, this method allows to have a very accurate and reliable evaluation.

Furthermore, in Table 1, as previously mentioned, data obtained from the analysis of vaginal fluid samples of women having vaginal pH ≥ 4.7 and sialidase or prolidase activity are reported.

As can be noted, in women having vaginal pH ≥ 4.7 for levels of sialidase activity comprised between +1 and +2, the OR values for LBW or for PTD are in the range from 1 to 2. Consequently, the risk for LBW and PTD is medium. Whereas, for levels of sialidase activity +3, the OR value for LBW is comprised between 2 and 4 and the OR value for PTD is comprised between 1 and 2. It follows that the risk for LBW is high, whereas the risk for PTD is medium. Finally, for levels of sialidase activity +4, the OR value for LBW and PTD is in the range from 2 to 4 so the corresponding risk is high.

In respect to levels of prolidase activity from +1 to +3 in samples of vaginal fluid with pH ≥ 4.7, the OR values for LBW and PTD are in the range from 1 to 2 so that the corresponding risk is medium. At variance, for levels of prolidase activity +4, the OR value is higher than 4 for LBW and in the range from 2 to 4 for PTD. Consequently, the risk for LBW is very high, whereas the risk for PTD is high.

According to an embodiment of the invention, the method can be carried out in samples of vaginal fluid with a pH equal or higher than 4.7.

Furthermore, in Table 1, as previously mentioned, data from the analysis of samples of vaginal fluid from women with BV and in which sialidase or prolidase activity was measured are reported.

In particular, in women with BV for levels of sialidase activity comprised between +1 and +2, the OR values for LBW are in the range from 1 to 2, whereas the OR values for PTD are below 1, so it follows that the risk for LBW is medium, whereas the risk for PTD is low or absent. For levels of sialidase activity comprised between +3 and +4, the OR values for LBW are in the range from 2 to 4, so it follows that the risk for LBW is medium.

Instead, as regards to PTD, levels equal or above +3 can be distinguished from levels equal or higher than +4. In the first case the OR value is between 1 and 2 and so the risk is medium, in the second case, the OR value is in the range from 2 to 4 and so the risk is high.

For levels of prolidase activity comprised between +1 and +3, the OR values for LBW are in the range from 1 to 2, so it follows that the risk for LBW is medium. Instead, for the same values of prolidase activity the OR values for PTD are below 1 and so the risk of PTD is low or absent.

It is to notice that for levels of prolidase activity equal or higher than +4 the OR value both for LBW and PTD is higher than 4 so that the risk in both cases is very high.

From what described, according to a first embodiment, the method of the invention for the determination of the risk of complications can be carried out in body fluid samples of women with BV.

Finally, in Table 1, data obtained from the analysis of vaginal fluid samples of women colonized by *G. vaginalis* and positive for sialidase or prolidase activity are shown.

In particular, for levels of sialidase activity comprised between +1 and +2, the OR values for LBW and for PTD are in the range from 1 to 2, so it follows that the risk is medium. For levels of sialidase activity equal or higher than +3 the OR value for LBW is in the range from 2 to 4, whereas the OR value for PTD is in the range from 1 to 2. It follows that the risk for LBW is high, whereas the risk for PTD is medium. For levels of sialidase activity equal or higher than +4 the OR values for LBW and for PTD are in the range from 2 to 4 and so for both the pregnancy adverse outcomes the risk is high.

Instead, for levels of prolidase activity comprised between +1 and +2, the OR values for LBW and PTD are in the range from 1 to 2, so it follows that the risk for LBW and PTD is medium. For levels of prolidase activity equal or higher than +3 the OR value for LBW is in the range from 2 to 4, so the corresponding risk is high, whereas the OR value for PTD is in the range from 1 to 2, so that the correlated risk is medium. Finally, for levels of prolidase activity equal or higher than +4 the OR value for LBW and for PTD is higher than 4 and so the correlated risk is very high.

From what above described, accordingly with a second embodiment, the method of the invention for the determination of the risk of complications can be carried out in body fluid samples of women colonized by *G. vaginalis.*

The advantage of these two embodiments of the current invention resides on the opportunity to select women at very high risk that from a simple determination of sialidase and prolidase activity result to have high risk, by a combined bacteriological analysis, or diagnosis of BV, or measurement of the vaginal pH.

As mentioned in the introductive part of the current description, the method of this invention also allows, profitably, to determine the risk of pathologies as acquisition of the HIV viral infection, HPV viral infection, upper genital tract infections (PID), post-partum or post-gynecologic surgery infections, spontaneous abortion, endometritis, cervitis.

In the following part an example regarding a study of acquisition of the HIV viral infection will be described.

For setting the method a group of 50 women at high risk of sexual acquisition of HIV infection was initially enrolled. Three years later, among these women a subgroup of 42 women were still HIV seronegative (controls) and a subgroup of 8 women became HIV seropositive.

These groups were subdivided according to the presence in the body fluid samples of the sialidase activity value, the prolidase activity value, a pH equal or higher than 4.7, diagnosis of bacterial vaginosis (BV), and presence of *G. vaginalis.*

Table 2 shows the values of the vaginal enzymatic activities found in the subgroup of the 42 HIV seronegative women (controls) and of the subgroup of the 8 women that became HIV seropositive.

**TABLE 2**

| | Positive % **OR** | | |
|---|---|---|---|
| **Measured parameter and/or vaginal colonization** | **Seronegative n=42** | **Seropositive n=8** | |
| **Sialidase activity** | | | |
| ≥ +1 | 64.3 | 75.0 | **1.3** |
| ≥ +2 | 40.5 | 62,5 | **1.6** |
| ≥ +3 | 14.3 | 50.0 | **3.6** |
| ≥ +4 | 9.5 | 37.5 | **4.2** |
| **Prolidase activity** | | | |
| ≥ +1 | 83.3 | 100.0 | **1.1** |
| ≥ +2 | 73.8 | 87.5 | **1.3** |
| ≥ +3 | 16.7 | 50.0 | **3.2** |
| ≥ +4 | 11.9 | 50.0 | **4.4** |
| **pH ≥ 4.7** | 81.0 | 87.5 | **1.2** |
| **pH ≥ 4.7 and Sialidase activity** | | | |
| ≥ +1 | 59.5 | 75.0 | **1.4** |
| ≥ +2 | 35.7 | 62,5 | **1.8** |
| ≥ +3 | 14.3 | 50.0 | **3.6** |
| ≥ +4 | 9.5 | 37.5 | **4.2** |
| **pH ≥ 4.7 and Prolidase activity** | | | |
| ≥ +1 | 73.8 | 87.5 | **1.3** |
| ≥ +2 | 71.4 | 87.5 | **1.4** |
| ≥ +3 | 14.3 | 50.0 | **3.6** |
| ≥ +4 | 11.9 | 50.0 | **4.4** |
| **BV** | 71.4 | 87.5 | **1.4** |
| **BV and Sialidase activity** | | | |
| ≥ +1 | 54.8 | 75.0 | **1.5** |
| ≥ +2 | 33.3 | 62.5 | **2.0** |
| ≥ +3 | 11.9 | 50.0 | **4.3** |
| ≥ +4 | 9.5 | 37.5 | **4.2** |
| **BV and Prolidase activity** | | | |
| ≥ +1 | 73.8 | 87.5 | **1.3** |
| ≥ +2 | 69.0 | 87.5 | **1.5** |
| ≥ +3 | 11.9 | 50.0 | **4.3** |
| ≥ +4 | 9.5 | 50.0 | **5.5** |
| ***G. vaginalis*** | 100.0 | 100.0 | **1.0** |
| ***G. vaginalis* and Sialidase activity** | | | |
| ≥ +1 | 64.3 | 75.0 | **1.3** |
| ≥ +2 | 40.5 | 62.5 | **1.6** |
| ≥ +3 | 14.3 | 50.0 | **3.6** |
| ≥ +4 | 9.5 | 37.5 | **4.2** |
| ***G. vaginalis* and Prolidase activity** | | | |
| ≥ +1 | 83.3 | 100.0 | **1.1** |
| ≥ +2 | 73.8 | 87.5 | **1.3** |
| ≥ +3 | 16.7 | 50.0 | **3.2** |
| ≥ +4 | 11.9 | 50.0 | **4.4** |

The main result shown in Table 2 is the OR value because it represents the index of the risk of acquisition of HIV infection by sexual transmission.

If this ratio is below 1 it means that the woman has a low or no risk of sexual acquisition of the viral infection. A ratio in the range from 1 to 2 is indicative of a medium risk of viral infection acquisition. If the ratio is comprised from 2 to 4 the women is at high risk of viral infection acquisition. Finally; if the ratio is over 4 it means that the woman is at very high risk of viral infection acquisition. In particular, and for example, a medium risk corresponding to an OR value of 2 means that the patient has twice the likelihood of viral infection acquisition in respect to normal women, or, in other words, a risk 100% higher.

Preferably, this result can be point out with a score: - meaning a low or no risk, + medium risk, ++ high risk, and +++ very high risk.

Values shown in Table 2 have the same meaning of values in Table 1 showing the data for LBW or PTD, so a detailed comment on will not made.

From Tables 1 and 2 it comes out that the OR value has an essentially increasing profile in relation to the increase of the levels of sialidase or prolidase activity.

From here on, an example of determination of the sialidase and prolidase activity in a sample of vaginal fluid will be reported at indicative and not limitative title of the current invention.

### DETERMINATION OF SIALIDASE ACTIVITY

50 µl of vaginal fluid sample are incubated for 90 min at room temperature with 50 µl of 2-(3'-methoxyphenyl)-N-acetyl-D-neuraminic acid (Sigma) substrate dissolved in sodium acetate 50 mM at pH = 5. Samples with sialidase activity develop a red color measurable at 492 nanometers, after addition of a developing solution of 4 mM 4-aminoantipyrine and 6 mM potassium ferricyanide. Results are expressed as difference between the absorbance of the sample after addition of the developing solution and that of a duplicate sample to which the developing reagents are not added. Specific activity was expressed by comparison with a standard curve carried out with known amounts of pure methoxyphenol.

Alternatively, the sialidase activity assay can be performed with chromogenic or fluorogenic substrates different from 2-(3'-methoxyphenyl)-N-acetyl-D-neuraminic acid (Sigma, M1393), as 2-O-(o-nitrophenyl)-alfa-D-N-acetyl neuraminic acid (Sigma, N1266, N1516), 2'-(4-methylumbelliferyl)-alfa-D-N-acetyl neuraminic acid sodium salt (Sigma, M8639), 5-bromo-4-chloro-3-indolyl-alfa-D-N-acetyl neuraminic acid (X-NeuNAc), (Sigma, B 4666; or Calbiochem 203770).

### DETERMINATION OF PROLIDASE ACTIVITY

50 µl of vaginal fluid sample are incubated for 24 hours at 37°C with 50 µl of L-proline-para-nitroanilide substrate (Sigma) dissolved in 100 mM sodium acetate at pH = 5. Samples with prolidase activity develop a yellow color measured at 405 nanometers. A duplicate sample without the substrate is used to subtract the sample background, whereas the spontaneous hydrolysis of the substrate is subtracted by the average value of two blanks without the vaginal fluid. Results are expressed as milli optical density units (mOD).

Alternatively, the assay of prolidase activity can be performed with chromogenic or fluorogenic substrates different from L-proline-para-nitroanilide (Sigma P5267) as: L-proline-beta-naphthylamide (Sigma P1380), N-benzyloxycarbonyl-L-prolyl-beta-naphthylamide, N-benzyloxycarbonyl-L-proline-para-nitrophenyl ester (Sigma C4877), hydroxy-L-prolyl-beta-naphthylamide, L-proline-7-amido-4-methyl-coumarin (Sigma P5898), L-proline-4-methoxy-beta-naphthylamide (Sigma P9655).

A kit for the determination of the risk of pathologies related to the presence of sialidase and/or prolidase activity in samples of vaginal fluid of women is described below.

Said kit can comprise a sialidase and/or prolidase activity assay in solution that includes a colorless substrate solution in which to inoculate the biologic sample; a developing solution in a container equipped with dispenser; a reference scale to evaluate the level of the enzymatic activity by the intensity of the developed color.

Said kit can also comprise a sialidase and/or prolidase activity test on solid support, as a platform, comprised of two membranes supported on a solid frame, and containing an enzyme substrate and an agent for the development of the color, through which the biological sample is passed; and a reference scale to evaluate the entity of the enzymatic activity by the intensity of the developed color.

Said kit can also comprise a sialidase and/or prolidase activity test on a solid support, comprising a membrane impregnated with a chromogenic or fluorogenic substrate of the enzyme and eventually an agent for the color development, supported on an inert strip, to be touched with the biological sample. Said kit will be furnished with a reference scale to evaluate the entity of the enzymatic activity by the intensity of the developed color.

Said kit can also comprise a pH indicator that is preferably a pH revealing paper with a turning interval in the range between 4 and 7.

It is to be noted that the kit can profitably be a combined kit. Said kit, for example, can comprise a test in solution or a test on solid support for the determination of the sialidase activity and/or the prolidase activity and a pH indicator.

It is now evident that, with reference to the evaluations contained in the above shown Tables and to the evaluations obtained from the described or anyhow available analytical tests, a kit that can provide in a fast way the determination of the risk of the above described pathologies is of major value to furnish the physician effective tools to decide about the pharmacological cure to administer to patients and to verify the efficacy of therapy.

As can be appreciated from what described, the method to determine the risk of pathologies as low birth weight (LBW), preterm birth (PTD), premature rupture of membranes, intraamniotic infections, spontaneous abortion, endometritis, post-partum and post-gynecologic surgery infections, upper genital tract infections (PID) which cause infertility, cervicitis, susceptibility to sexually transmitted diseases as viral infections from papillomavirus (HPV) and human immunodeficiency virus (HIV), and vertical transmission of HIV infection, accordingly to this invention, allows to fulfill the demands described in the introductive part of the current invention.

## Claims

1. Method for the determination of the risk of pathologies related to the presence of sialidase and/or prolidase activity consisting of low birth weight (LBW), preterm birth (PTD), premature rupture of membranes, intraamniotic infections, spontaneous abortion endometritis, post-partum and post-gynecologic surgery infections, upper genital tract infections which cause infertility, cervicitis, susceptibility to sexually transmitted diseases as viral infections from papillomavirus (HPV) and human immunodeficiency virus (HIV), and vertical transmission of HTV infection, in vaginal fluid samples of women, comprising the following steps in order:
a) determination of the levels of sialidase and/or prolidase activity in said sample of vaginal fluid;
b) comparison of said levels of sialidase and/or prolidase activity with ranges of prefixed values of said activity;
c) calculation of the risk factor.

2. Method as set forth in claim 1, in which after the a) step a score of said levels of sialidase and/or prolidase activity is determined.

3. Method as set forth in claim 1 or 2, in which the risk of pathologies is performed in samples of vaginal fluid with pH equal or higher than 4.7.

4. Method as set forth in claim 1 or 2, in which the risk of pathologies is performed in samples of vaginal fluid of women with BV.

5. Method as set forth in claim 1 or 2, in which the risk of pathologies is performed in samples of vaginal fluid of women colonized by the *Gardnerella vaginalis* bacterium.

6. Method as set forth in any one of claims from 1 to 5, in which said method is carried out in samples of vaginal fluid of women between the first or second trimester of gestation.

7. Method as set forth in any one of claims from 1 to 6, in which said method is carried out in samples of vaginal fluid of women from the seventh to the twenty-fourth full week of gestation.

8. Method as set forth in any one of claims from 1 to 7, in which said method is carried out in samples of vaginal fluid of pregnant women.

## Patentansprüche

1. Verfahren zur Bestimmung des Risikos von Krankheiten in Zusammenhang mit der Anwesenheit von Sialidase- und/oder Prolidaseaktivität, bestehend aus niedrigem Geburtsgewicht (LBW, engl. "low birth weight"), Frühgeburt (PTD, engl. "preterm birth"), vorzeitigem Blasensprung, intraamniotischen Infektionen, Spontanabort, Endometritis, nachgeburtlichen Infektionen und Infektionen nach gynäkologischen Operationen, Infektionen des oberen Genitaltrakts, die Unfruchtbarkeit verursachen, Cervicitis, Anfälligkeit für sexuell übertragbare Krankheiten wie virale Infektionen durch Papillomavirus (HPV) und humanes Immundefizienzvirus (HIV) und vertikale Übertragung von HIV-Infektion, in Proben von Vaginalsekret von Frauen, umfassend die folgenden Schritte in der Reihenfolge:
a) Bestimmung der Niveaus von Sialidase- und/oder Prolidaseaktivität in besagter Probe von Vaginalsekret;
b) Vergleich der besagten Niveaus von Sialidase- und/oder Prolidaseaktivität mit Bereichen von vorbestimmten Werten der besagten Aktivität.
c) Berechnung des Risikofaktors.

2. Verfahren wie in Anspruch 1 dargelegt, wobei nach dem Schritt a) ein Punktwert der besagten Niveaus von Sialidase- und/oder Prolidaseaktivität bestimmt wird.

3. Verfahren wie in Anspruch 1 oder 2 dargelegt, wobei das Krankheitsrisiko in Vaginalsekretproben mit pH-Werten gleich oder größer als 4,7 bestimmt wird.

4. Verfahren wie in Anspruch 1 oder 2 dargelegt, wobei das Krankheitsrisiko in Vaginalsekretproben von Frauen mit BV bestimmt wird.

5. Verfahren wie in Anspruch 1 oder 2 dargelegt, wobei das Krankheitsrisiko in Vaginalsekretproben von Frauen, die von dem Bakterium *Gardnerella vaginalis* besiedelt sind, bestimmt wird.

6. Verfahren wie in einem der Ansprüche 1 bis 5 dargelegt, wobei besagtes Verfahren in Vaginalsekretproben von Frauen in dem ersten oder zweiten Schwangerschaftstrimester durchgeführt wird.

7. Verfahren wie in einem der Ansprüche 1 bis 6 dargelegt, wobei besagtes Verfahren in Vaginalsekretproben von Frauen von der siebten bis zur vierundzwanzigsten vollen Schwangerschaftswoche durchgeführt wird.

8. Verfahren wie in einem der Ansprüche 1 bis 7 dargelegt, wobei besagtes Verfahren in Vaginalsekretproben von schwangeren Frauen durchgeführt wird.

## Revendications

1. Procédé de détermination du risque de pathologies associées à la présence d'activité sialidase et/ou prolidase, consistant en un faible poids à la naissance (FPN), une naissance avant terme, une rupture prématurée des membranes, des infections intra-amniotiques, un avortement spontané, une endométrite, des infections post-partum et subséquentes à une chirurgie gynécologique, des infections des voies génitales supérieures qui provoquent la stérilité, une cervicite, une sensibilité aux maladies sexuellement transmissibles telles que des infections virales provenant du papillomavirus (HPV) et du virus de l'immunodéficience humaine (VIH), et une transmission verticale d'une infection au VIH, dans des échantillons de fluides vaginaux de femmes, comprenant les étapes suivantes dans l'ordre:
a) la détermination des taux d'activité sialidase et/ou prolidase dans ledit échantillon de fluide vaginal ;
b) la comparaison desdits taux d'activité sialidase et/ou prolidase avec des plages de valeurs préfixées de ladite activité ;
c) le calcul du facteur de risque.

2. Procédé selon la revendication 1, dans lequel après l'étape a), un score desdits taux d'activité sialidase et/ou prolidase est déterminé.

3. Procédé selon la revendication 1 ou 2, dans lequel le risque de pathologies est déterminé dans des échantillons de fluide vaginal avec un pH égal ou supérieur à 4,7.

4. Procédé selon la revendication 1 ou 2, dans lequel le risque de pathologies est déterminé dans des échantillons de fluide vaginal de femmes souffrant de VB.

5. Procédé selon la revendication 1 ou 2, dans lequel le risque de pathologies est déterminé dans des échantillons de fluide vaginal de femmes colonisées par la bactérie *Gardnerella vaginalis.*

6. Procédé selon l'une quelconque des revendications 1 à 5, où ledit procédé est réalisé dans des échantillons de fluide vaginal de femmes entre les premier et deuxième trimestres de la grossesse.

7. Procédé selon l'une quelconque des revendications 1 à 6, où ledit procédé est réalisé dans des échantillons de fluide vaginal de femmes de la septième à la vingt-quatrième semaine pleine de grossesse.

8. Procédé selon l'une quelconque des revendications 1 à 7, où ledit procédé est réalisé dans des échantillons de fluide vaginal de femmes enceintes.
